# FASCICULE DE BREVET EUROPEEN

(11) **EP 1 040 813 B1**
(45) Date de publication et mention de la délivrance du brevet: **06.02.2002**
(21) Numéro de dépôt: 00400692.0
(22) Date de dépôt: 13.03.2000
(51) Int. Cl.: A61K 7/043

(54) **Vernis à ongles à effet martelé**
Nagellack mit Hammerschlageffekt
Nail polish with hammer stroke effect

(30) Priorité: 31.03.1999 FR 9904047
(43) Date de publication de la demande: 04.10.2000
(73) Titulaire: L'OREAL, 75008 Paris (FR)
(72) Inventeur: Ramin, Roland, 75014 Paris (FR); Brenne, Ingrid, 94240 L'Hay-Les-Roses (FR)
(74) Mandataire: Kromer, Christophe

(56) Documents cités:
- EP-A- 0 705 594
- EP-A- 0 745 372
- EP-A- 0 797 977
- EP-A- 0 819 420
- FR-A- 2 578 741
- US-A- 2 884 388
- DATABASE WPI Week 199644 Derwent Publications Ltd., London, GB; AN 1996-438259 XP002126526 & JP 08 216331 A (NISSIN STEEL CO. LTD.), 27 août 1996 (1996-08-27)

## Description

La présente invention a pour objet une composition de vernis à ongles comprenant dans un milieu solvant organique un polymère filmogène, de la silice pyrogénée, un pigment métallique et un organopolysiloxane. L'invention a aussi pour objet un procédé de maquillage des ongles, notamment des ongles d'êtres humains ou des faux ongles.

Les compositions de vernis à ongles comprennent généralement un polymère filmogène soit solubilisé dans un solvant organique, soit dispersé sous forme de particules dans un milieu aqueux, et une matière colorante, notamment un pigment. De tels vernis sont par exemple décrits dans les documents US-A-4158053 et FR-A-2578741.

Ces vernis à ongles, après l'application sur l'ongle d'une ou plusieurs couches puis séchage, conduisent généralement à la formation d'un film lisse, continu et homogène, brillant ou mat. Certains des films obtenus présentent aussi de bonnes propriétés cosmétiques telles qu'une bonne tenue et en particulier une bonne adhérence sur l'ongle, une bonne résistance à l'eau, aux frottements et aux chocs.

Avec l'évolution de la mode, les consommateurs, de plus en plus exigeants, recherchent de nouveaux produits de maquillage conférant des effets de maquillage originaux ou particuliers. Un besoin subsiste donc de disposer de produit de maquillage dont l'application sur un support comme les ongles, la peau ou les cheveux d'êtres humains conduit à un effet de maquillage différent de ceux des films lisses, continus et homogènes actuellement obtenus avec les produits disponibles sur le marché.

Par ailleurs, il est connu du document US-A-2884388 une composition de revêtement de surface produisant un film d'aspect martelé sur le support où il est appliqué tel qu'une surface métallique, du bois ou du papier. L'aspect martelé correspond à l'aggrégation de pigment métallique à la surface du film sous la forme de petits cercles proéminents, régulièrement répartis et présentant l'aspect de cratères. Cet aspect est obtenu avec une composition comprenant dans un milieu solvant organique un polymère filmogène, un pigment métallique et un composé siliconé particulier. La composition est appliquée par vaporisation sous forme de spray pour obtenir l'effet martelé recherché.

Les compositions de vernis à ongles connues sont habituellement appliquées à l'aide d'un pinceau et présentent une certaine consistance (et notamment de la thixotropie) pour faciliter l'application et assurer une bonne dispersion des pigments colorés dans le milieu. Ainsi, il est connu du document GB-A-2021411 d'utiliser des argiles telles que les montmorillonites organomodifiées pour obtenir un vernis de la consistance voulue.

En utilisant le composé siliconé et le pigment métallique, selon le document US-A-2884388, dans une composition de vernis à ongles comprenant de la montmorillonite comme unique agent de consistance, le demandeur a constaté que le film obtenu, après application sur les ongles, ne présentait pas d'aspect martelé. Le film reste lisse et continu comme pour un vernis à ongles classique.

Il est par ailleurs connu du document EP-A-745372 un vernis à ongles contenant de l'acide silicique colloïdal pour renforcer les ongles. Le document EP-A-797977 décrit un vernis à ongles comprenant un mélange de polymère filmogène.

Le but de la présente invention est de proposer une composition de vernis à ongles fluide et facile à appliquer conduisant, après application, à un aspect martelé bien régulier et bien visible à l'oeil.

Le demandeur a découvert, de façon surprenante, qu'un nouveau type de maquillage des ongles pouvait être obtenu en utilisant une composition filmogène comprenant de la silice pyrogénée, un pigment métallique et un composé siliconé particulier.

De façon plus précise, la présente invention a pour objet une composition de vernis à ongles comprenant dans un milieu solvant organique au moins un polymère filmogène, caractérisée par le fait qu'elle comprend au moins une silice pyrogénée, au moins un pigment métallique et au moins un organopolysiloxane particulier.

Un autre objet de l'invention est un procédé de maquillage des ongles consistant à appliquer sur les ongles la composition telle que définie précédemment.

Lorsque la composition est appliquée sur les ongles, des cratères se forment à la surface du film, pendant le séchage, provoquant la migration du pigment métallique sur le bord des cratères. Les cratères présentent des tailles différentes et ont un aspect plus ou moins régulier, notamment en forme de cercle ou d'ovoïde. Les cratères peuvent présenter un faible relief ou bien un relief pentu similaire à un cône. La pointe du cône peut être ouverte et laisser apparaître le support sur lequel est appliqué la composition. Après séchage complet, le film présente un aspect martelé.

La silice pyrogénée présente dans la composition, en plus de sa fonction d'agent de consistance et d'agent rhéologique, ne nuit pas à la formation de l'aspect martelé contrairement à la montmorillonite utilisée seule. On obtient ainsi un maquillage original présentant une bonne tenue, notamment aux frottements et à l'écaillage, ainsi qu'une bonne adhérence sur l'ongle.

Avantageusement cette composition peut être appliquée comme produit de surface, communément appelée "top coat" en terminologie anglosaxonne.

Un autre objet de l'invention est un procédé cosmétique de maquillage des ongles consistant à appliquer sur les ongles une composition telle que définie précédemment.

L'invention a aussi pour objet un procédé cosmétique de maquillage des ongles consistant à appliquer sur les ongles une première couche, appelée aussi couche de base, d'une première composition cosmétique comprenant dans un milieu cosmétiquement acceptable un premier polymère filmogène, puis à appliquer sur au moins une partie de la première couche une deuxième couche, appelée aussi couche de surface, d'une deuxième composition cosmétique comprenant, dans un milieu solvant organique, au moins un deuxième polymère filmogène, au moins un pigment métallique et au moins un organopolysiloxane, la première composition ne contenant pas d'organopolysiloxane comme présent dans la deuxième composition.

L'invention a également pour objet un kit de maquillage comprenant :
- une première composition cosmétique (de couche de base) comprenant dans un milieu cosmétiquement acceptable au moins un premier polymère filmogène ;
- une deuxième composition cosmétique (de couche de surface) comprenant, dans un milieu solvant organique, au moins un deuxième polymère filmogène, au moins une silice pyrogénée, au moins un pigment métallique et au moins un organopolysiloxane, la première composition ne contenant pas d'organopolysiloxane comme présent dans la deuxième composition.

L'invention a encore pour objet des faux-ongles maquillés comprenant un maquillage susceptible d'être obtenu selon le procédé tel que défini précédemment.

L'organopolysiloxane présent dans la composition de surface a une viscosité telle qu'une solution à 10 % d'organopolysiloxane dans le xylène a une viscosité, à 25 °C, allant de 0,0002 m²/s à 0,01 m²/s (20 à 1000 centistokes), de préférence de 0,0005 m²/s à 0,005 m²/s (50 à 500 centistokes), et mieux de 0,001 m²/s à 0,0015 m²/s (100 à 150 centistokes). La viscosité est mesurée selon la norme ASTM D-445 avec un tube capillaire en verre.

L'organopolysiloxane a en moyenne de 1,99 à 2,01 radicaux hydrocarbonés par atome de silicium. Au moins 50 % des unités de l'organopolysiloxane sont des unités de formule RMeSiO dans laquelle R désigne un radical aliphatique ayant de 1 à 3 atomes de carbone, les autres unités restantes étant des unités de formule R'ₙSiO_{(2-n/2)} dans laquelle n a une valeur moyenne allant de 1 à 3 et R' désigne un radical hydrocarboné monovalent. Dans la composition selon l'invention, le rapport pondéral silice/polymère filmogène est différent de 0,0116.

R peut désigner notamment un radical méthyle, éthyle, propyle, vinyle ou allyle. L'organopolysiloxane peut être choisi parmi les polydiméthylsiloxanes, les polyéthylméthylsiloxanes, les polyméthylvinylsiloxanes, les polyméthylpropylsiloxanes, les polyméthylallylsiloxanes, les phénylméthylpolysiloxanes et leurs copolymères,

Le radical monovalent hydrocarboné R' peut désigner un radical alkyle en C₁-C₁₈ et notamment un radical méthyle, éthyle, propyle ou octadécyle ; un radical alcényle en C₁-C₆, notamment vinyle, allyle ou hexényle ; un radical hydrocarboné cycloaliphatique en C₆-C₁₂, notamment un radical cyclohexyle, cyclohexényle ; un radical hydrocarboné aralkyl, en C₇- C₁₅, notamment un radical benzyle ; un radical aromatique hydrocarboné en C₆-C₂₀, notamment phényle, tolyle, xényle, naphthyle.

De préférence, l'organopolysiloxane peut être choisi parmi les polydiméthylsiloxanes, les polyméthyléthylsiloxanes, les copolymères de diméthylsiloxane et de méthylvinylsiloxane, les copolymères de diméthylsiloxane et jusqu'à 50 moles pourcent de phénylméthylsiloxane.

Avantageusement, on peut utiliser un polydiméthylsiloxane.

L'organopolysiloxane peut être présent dans la composition de surface en une teneur efficace pour obtenir l'effet martelé (formation de cratère) et notamment en une teneur allant de 0,0001 % à 1,25 % en poids, par rapport au poids total de la composition, de préférence de 0,001 % à 0,5 % en poids, et mieux de 0,001 % à 0,25 % en poids.

Comme organopolysiloxane, on peut utiliser ceux vendus en solution dans du xylène sous la dénomination "Dow Corning® 61 additive" par la Société Dow Corning, ou bien encore ceux commercialisés sous la dénomination "Rhodorsil® SIL AID 16" par la Société Rhône-Poulenc.

Pour obtenir un film présentant l'aspect martelé, la composition de surface doit comprendre au moins un pigment métallique. Comme pigment métallique, on peut utiliser par exemple une poudre d'aluminium, de cuivre, de zinc, de bronze, de nickel, de chrome, ou équivalents.

Le pigment métallique peut être enrobé, par exemple par de la colophane ou des acides gras comme l'acide oléique ou linoléique.

Le pigment métallique peut avoir une taille moyenne de particules allant de 5 à 150 µm, et de préférence de 10 à 60 µm.

Comme pigment métallique, on peut utiliser les poudres d'aluminium vendues sous les dénominations "STAPA Non leafing grade", "STAPA Metallic", "METALURE L55350" par la Société ECKART, "Aluminium Super 800" par la Société WOLSTENHOLME International, "SILVET ET 1630" par la Société SILBERLINE ; les poudres de bronze vendues sous les dénominations "STAPA Golden Bronze" par la Société ECKART, "Pastel Standard" par la Société WOLSTENHOLME International.

Le pigment métallique peut être présent dans la composition de surface en une teneur allant de 0,1 % à 25 % en poids, par rapport au poids total de la composition, de préférence de 1 % à 10 % en poids, et mieux de 1 % à 5 % en poids.

De préférence, l'organopolysiloxane et le pigment métallique peuvent être présents dans la composition de surface en un rapport pondéral organopolysiloxane/pigment métallique allant de 0,1/100 à 5/100, et mieux de 0,5/100 à 1,5/100. On obtient dans ce rapport pondéral un film présentant un bon effet martelé.

Selon l'invention, le milieu cosmétiquement acceptable de la composition de base peut comprendre un milieu aqueux ou un milieu solvant organique.

Dans la présente demande, on entend par "polymère filmogène", un polymère apte à former à lui seul ou en présence d'un agent plastifiant, un film isolable. Le polymère filmogène de la composition de base peut être solubilisé ou dispersé sous forme de particules dans le milieu cosmétiquement acceptable de la composition.

Le polymère filmogène peut être choisi notamment parmi les polymères radicalaires, les polycondensats et les polymères d'origine naturelle, et de préférence dans le groupe formé par les polymères vinyliques, les polyuréthanes, les polyesters, les résines alkydes, les résines époxyesters, les polymères cellulosiques tels que la nitrocellulose, les esters de cellulose, les résines résultant de la condensation de formaldéhyde avec une arylsulfonamide, et leurs mélanges.
Le polymère filmogène est de préférence solubilisé dans un milieu solvant organique.

De préférence, le polymère filmogène de la composition de surface peut comprendre au moins un ester de cellulose. On a en effet constaté que l'ester de cellulose fige rapidement les cratères formés lors de l'application de la composition de surface sur les ongles et conduit à un maquillage présentant un bel effet martelé.

L'ester de cellulose utilisé selon l'invention peut comprendre des groupements acyle R-CO- dont le groupement R désigne un radical alkyle, linéaire ou ramifié, ayant de 1 à 3 atomes de carbones, ou leurs mélanges.

En particulier, l'ester de cellulose peut être choisi parmi l'acétate de cellulose, l'acétopropionate de cellulose, l'acétobutyrate de cellulose, de préférence parmi l'acétobutyrate de cellulose et l'acétopropionate de cellulose, et mieux l'acétobutyrate de cellulose.

L'acétobutyrate de cellulose peut comprendre un taux pondéral en groupement acétate allant de 1 à 18 % et un taux pondéral en groupement butyrate allant de 30 à 60 %. Comme acétobutyrate de cellulose (dit ABC), on peut utiliser ceux vendus sous les dénominations "CAB-551", "CAB-500", "CAB 553", "CAB-381"par la Société Eastman Chemical.

Avantageusement, le polymère filmogène présent dans la composition de surface peut comprendre un mélange de nitrocellulose et d'ester de cellulose tel que décrit précédemment.

La nitrocellulose peut être choisie parmi la nitrocellulose RS 1/8 sec ; RS ¼ sec. ; RS ½ sec. ; RS 5 sec. ; RS 15 sec. ; RS 35 sec. ; RS 75 sec.; RS 150 sec. ; AS ¼ sec. ; AS ½ sec. ; SS ¼ sec. ; SS ½ sec. ; SS 5 sec., notamment commercialisée par la Société HERCULES.

De préférence, les compositions de base et/ou de surface peuvent comprendre de 0,05 % à 30 % en poids de nitrocellulose et de 0,05 % à 30 % en poids d'ester de cellulose, notamment d'acétobutyrate de cellulose, par rapport au poids total de la composition. Avantageusement, l'ester de cellulose peut être présent dans les compositions en une teneur pondérale allant de 10 % à 80 % en poids par rapport au poids total d'ester de cellulose et de nitrocellulose présents dans la composition.

Selon une variante de réalisation selon l'invention, la composition de base peut comprendre un milieu aqueux. Le polymère filmogène est généralement présent sous forme de particules en dispersion dans le milieu aqueux.

Comme polymère en dispersion aqueuse, on peut citer les dispersions de polymères acryliques vendues sous les dénominations NEOCRYL XK-90® , NEOCRYL A-1070® , NEOCRYL A-1090® , NEOCRYL BT-62® , NEOCRYL A-1079® , NEOCRYL A-523® par la Société ZENECA, DOW LATEX 432® par la Société DOW CHEMICAL. On peut également employer des dispersions aqueuses de polyuréthane, et notamment les polyester-polyuréthanes vendus sous les dénominations "AVALURE UR-405® ", "AVALURE UR-410® ", "AVALURE UR-425® ", "SANCURE 2060® " par la Société GOODRICH et les polyéther-polyuréthanes vendus sous les dénominations "SANCURE 878® " par la Société GOODRICH, "NEOREZ R 970® " par la Société ICI.

Le polymère filmogène des compositions de base et de surface peut être présent selon l'invention en une teneur, en matières sèches, efficace pour l'obtention d'un film, notamment en une teneur allant de 1 % à 60 % en poids, par rapport au poids total de la composition, et mieux de 5% à 45 % en poids.

Comme solvant organique utilisable dans l'invention, on peut citer :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde
- leurs mélanges.

On peut utiliser de préférence comme solvant organique l'acétate de butyle, l'acétate d'éthyle, l'alcool isopropylique et leurs mélanges.

Dans chaque composition à milieu solvant organique, le solvant organique peut être présent en une teneur allant de 25 à 95 % en poids, par rapport au poids total de la composition, et de préférence de 60 % à 80 % en poids.

Lorsque la composition de base selon l'invention contient un milieu aqueux, ce dernier peut être constitué essentiellement d'eau ou bien encore d'un mélange hydroalcoolique et en particulier contenant des monoalcools inférieurs en C₁-C₅. La teneur en eau dans la composition à milieu aqueux peut aller de 30 à 95 % en poids, par rapport au poids total de chaque composition, et de préférence de 60 % à 90 % en poids.

La silice pyrogénée présente dans la composition de surface peut se présenter sous forme de silice pyrogénée hydrophile ou de silice pyrogénée hydrophobe. On utilise de préférence la silice pyrogénée hydrophile.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leurs surfaces. De telles silices hydrophiles sont par exemple commercialisées sous les dénominations "AEROSIL 130® ", "AEROSIL 200® ", "AEROSIL 255® ", "AEROSIL 300® ", "AEROSIL 380® " par la Société Degussa, "CAB-O-SIL HS-5® ", "CAB-O-SIL EH-5® ", "CAB-O-SIL LM-130® ", "CAB-O-SIL MS-55® ", "CAB-O-SIL M-5® " par la Société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812® " par la Société Degussa, "CAB-O-SIL TS-530® " par la Société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972® ", "AEROSIL R974® " par la Société Degussa, "CAB-O-SIL TS-610® ", "CAB-O-SIL TS-720® " par la Société Cabot.

La silice pyrogénée présente de préférence une taille de particules pouvant être nanométrique à micrométrique, par exemple allant d'environ 5 à 200 nm.

La silice pyrogénée peut être présente dans la composition de surface selon l'invention en une quantité efficace pour obtenir l'effet martelé, notamment en une teneur allant de 0,1 % à 5 % en poids, par rapport au poids total de la composition, de préférence de 0,5 % à 2 % en poids.

Il est également possible d'introduire dans la composition de surface une argile comme les bentonites organomodifiées, sans nuire à la propriété du film, Cette argile peut être présente en une quantité allant de 0,1 % à 3 % en poids, par rapport au poids total de la composition, et mieux de 0,5 % à 1,5 % en poids et dans un rapport pondéral argile/silice pyrogénée allant de 0 à 0,5.

Comme bentonites, on peut utiliser celles vendues sous les dénominations "Bentone 27® ", "Bentone 34® ", "Bentone 38® " par la Société Rheox, ou encore sous la dénomination "Tixogel LG® " par la Société Sud Chemie.

Pour améliorer les propriétés filmogènes de la composition, notamment de la composition de base et/ou de surface selon l'invention, un agent auxiliaire de filmification peut être prévu.

Un tel agent auxiliaire de filmification peut être choisi parmi tous les composés connus de l'homme du métier comme étant susceptibles de remplir la fonction recherchée, et être notamment choisi parmi les agents plastifiants,

En outre, lorsque la composition de base selon l'invention comprend un polymère filmogène sous forme de particules dispersées dans un milieu aqueux, l'agent auxiliaire de filmification peut aussi être choisi parmi les agents de coalescence.

Selon un mode préféré de réalisation du kit et du procédé de maquillage selon l'invention, la composition de base peut être apte à former un film de couleur différente de celle du film martelé obtenu avec la composition de surface. Err particulier, lorsque le film martelé comporte des cratères présentant des ouvertures (c'est-à-dire lorsque la pointe du cône du cratère est ouverte), les ouvertures peuvent laisser apparaître la couleur de la couche de base. On observe alors un contraste entre la couleur de la couche de base et la couleur de la couche de surface martelée. Le film de maquillage présente alors un effet de couleur original.

Les compositions de base et de surface selon l'invention peuvent par ailleurs contenir des adjuvants couramment utilisés dans les vernis à ongles. Parmi ces adjuvants, on peut citer les agents épaississants, les agents d'étalement, les agents mouillants, les agents dispersants, les anti-mousses, les conservateurs, les filtres UV, les colorants, les pigments différents des pigments métalliques cités précédemment, les actifs, les tensioactifs, les agents hydratants, les parfums, les neutralisants, les stabilisants, les antioxydants.

Bien entendu l'homme du métier veillera à choisir ce ou ces éventuels composés complémentaires, et/ou leurs quantités, de manière telles que les propriétés avantageuses de la composition selon l'invention ne soient pas, ou substantiellement pas, altérées par l'adjonction envisagée.

La composition de base et celle de surface selon l'invention peuvent être préparées par l'homme du métier sur la base de ses connaissances générales et selon l'état de la technique.

Avantageusement, les compositions de base et celle de surface peuvent être conditionnées dans des compartiments ou récipients distincts, accompagnés de moyens d'application appropriés, identiques ou différents, tels que des pinceaux, des brosses, des plumes, des éponges.

La composition de surface peut être appliquée soit à l'une des extrémités de la couche de base, soit au milieu, ou encore de façon discontinue notamment sous forme de motifs géométriques, symétriques ou dissymétriques (par exemple sous forme de points, carrés, ronds, étoiles), répartis de façon aléatoire ou ordonnée, à contours précis ou flous.

L'invention est illustrée plus en détail dans les exemples suivants.

### Exemple 1 :

On a préparé un vernis à ongles ayant la composition suivante :
- Nitrocellulose 15 g
- Acétobutyrate de cellulose (CAB 381-05 d'Eastman Chemical) 0,5 g
- Résine alkyde 9 g
- Silice pyrogénée (Aérosil 200 de DEGUSSA) 1 g
- Polydiméthylsiloxane en solution à 10 % dans le xylène (Dow Corning 61 Additive) 0,3 g
- Poudre d'aluminium enrobée de collophane (SILVET ET1630 de Silberline) 3 g
- Plastifiants 6 g
- Hectorite 0,3 g
- Pigment 0,2 g
- Solvants (acétate d'éthyle, acétate de butyle) qsp 100 g

En applicant le vernis sur les ongles, on a constaté la formation instantanée de petits cratères à la surface du film procurant un maquillage à effet martelé.

### Exemple 2 :

On a préparé deux compositions A et B de vernis à ongles suivantes :

### Composition de base A :

- Nitrocellulose 15 g
- Résine alkyde 9 g
- Plastifiant 6 g
- Hectorite 1,5 g
- Pigments rouges 1,5 g
- Acétate d'éthyle, acétate de butyle qsp 100 g

### Composition de surface B :

- Nitrocellulose 15 g
- Acétobutyrate de cellulose (CAB 381-05 d'Eastman Chemical) 0,5 g
- Résine alkyde 9 g
- Silice pyrogénée (Aérosil 200 de DEGUSSA) 1 g
- Polydiméthylsiloxane en solution à 10 % dans le xylène (Dow Coming 61 Additive) 0,1 g
- Poudre d'aluminium enrobée de collophane (SILVET ET1630 de Silberline) 1 g
- Plastifiants 6 g
- Hectorite 0,3 g
- Pigment 0,2 g
- Solvants (acétate d'éthyle, acétate de butyle) qsp 100 g

On a appliqué sur l'ongle une couche de base de la composition A puis, après séchage, une couche de surface de la composition B. On a obtenu un maquillage à effet martelé. Les cratères de la couche de surface présentent des ouvertures laissant apparaître la couleur rouge du film de la couche de base.
Le maquillage obtenu a une couleur globale rose parme.

### Example 3 :

On a préparé un vernis à ongles ayant la composition suivante :
- Nitrocellulose 15 g
- Acétopropionate de cellulose (CAP 482-0,5 d'Eastman Chemical) 0,5 g
- Résine alkyde 9 g
- Silice pyrogénée (Aérosil 200 de DEGUSSA) 1 g
- Polydiméthylsiloxane en solution à 10 % dans le xylène (Dow Corning 61 Additive) 0,1 g
- Poudre de bronze (Pastel Standard de Wolstenholme International) 1 g
- Plastifiants 6 g
- Solvants (acétate d'éthyle, acétate de butyle) qsp 100 g

En application le vernis sur les ongles, on a constaté la formation instantanée de cratères à la surface du film procurant un maquillage à effet martelé.

### Exemple 4 comparatif : (hors invention)

On a préparé un vernis à ongles ne faisant pas partie de l'invention ayant la composition suivante :
- Nitrocellulose 15 g
- Acétobutyrate de cellulose (CAB 381-05 d'Eastman Chemical) 0,5 g
- Résine alkyde 9 g
- Polydiméthylsiloxane en solution à 10 % dans le xylène (Dow Corning 61 Additive) 0,3 g
- Poudre d'aluminium enrobée de collophane (SILVET ET1630 de Silberline) 3 g
- Plastifiants 6 g
- Hectorite 1,5 g
- Pigment 0,2 g
- Solvants (acétate d'éthyle, acétate de butyle) qsp 100 g

La composition ne comprend que de l'hectorite comme agent épaississant. En application sur les ongles, on a constaté que le vernis ne forme pas de cratère ; le film reste lisse et continue.

## Revendications

1. Composition de vernis à ongles comprenant dans un milieu solvant organique au moins un polymère filmogène, **caractérisée par le fait qu'**elle comprend au moins une silice pyrogénée, au moins un pigment métallique et au moins un organopolysiloxane ayant en moyenne de 1,99 à 2,01 radicaux hydrocarbonés par atome de silicium, au moins 50 % des unités de l'organopolysiloxane étant des unités de formule RMeSiO dans laquelle R désigne un radical aliphatique ayant de 1 à 3 atomes de carbone, les autres unités restantes étant des unités de formule R'ₙSiO_{(2-n/2)} dans laquelle n a une valeur moyenne allant de 1 à 3 et R' désigne un radical hydrocarboné monovalent, une solution à 10 % en poids de l'organopolysiloxane dans le xylène ayant une viscosité, à 25 °C, allant de 0,0002 m²/s à 0,01 m²/s, le rapport pondéral silice/polymère filmogène étant différent de 0,0116.

2. Composition selon la revendication 1, **caractérisée par le fait que** la solution d'organopolysiloxane à 10 % en poids dans le xylène a une viscosité allant de 0,0005 à 0,005 m²/s, et mieux de 0,001 m²/s à 0,0015 m²/s.

3. Composition selon la revendication 1 ou 2, **caractérisée par le fait que** R désigne un radical méthyle.

4. Composition selon l'une quelconque des revendications précédentes, **caractérisée par le fait que** l'organopolysiloxane est choisi dans le groupe formé par les polydiméthylsiloxanes, les polyméthyléthylsiloxanes, les copolymères de diméthylsiloxane et de méthylvinylsiloxane, les copolymères de diméthylsiloxane et de phénylméthylsiloxane.

5. Composition selon l'une quelconque des revendications précédentes, **caractérisée par le fait que** l'organopolysiloxane est un polydiméthylsiloxane.

6. Composition selon l'une quelconque des revendications précédentes, **caractérisée par le fait que** l'organopolysiloxane est présent en une teneur allant de 0,0001 % à 1,25 % en poids, par rapport au poids total de la composition, de préférence de 0,001 % à 0,5 % en poids, et mieux de 0,001 % à 0,25 % en poids.

7. Composition selon l'une quelconque des revendications précédentes, **caractérisée par le fait que** le polymère filmogène est choisi dans le groupe formé par les polymères radicalaires, les polycondensats et les polymères d'origine naturelle, et de préférence dans le groupe formé par les polymères vinyliques, les polyuréthanes, les polyesters, les polymères cellulosiques.

8. Composition selon l'une quelconque des revendications précédentes, **caractérisée par le fait que** le polymère filmogène est présent en une teneur allant de 1 % à 60 % en poids, par rapport au poids total de la composition, et mieux de 5 % à 45 % en poids.

9. Composition selon l'une quelconque des revendications précédentes, **caractérisée par le fait que** le polymère filmogène comprend un ester de cellulose.

10. Composition selon l'une quelconque des revendications précédentes, **caractérisée par le fait que** le polymère filmogène comprend un ester de cellulose choisi dans le groupe formé par l'acétobutyrate de cellulose et l'acétopropionate de cellulose.

11. Composition selon la revendication 9 ou 10, **caractérisée par le fait que** l'ester de cellulose est présent en une teneur allant de 0,05 % à 30 % en poids, par rapport au poids total de la composition.

12. Composition selon l'une quelconque des revendications précédentes, **caractérisée par le fait que** le polymère filmogène comprend de la nitrocellulose.

13. Composition selon la revendication précédente, **caractérisée par le fait que** le nitrocellulose est présent en une teneur allant de 0,05 % à 30 % en poids, par rapport au poids total de la composition.

14. Composition selon l'une quelconque des revendications précédentes, **caractérisée par le fait que** le pigment métallique est choisi dans le groupe formé par la poudre d'aluminium, de cuivre, de zinc, de bronze, de nickel, de chrome, et leurs mélanges.

15. Composition selon l'une quelconque des revendications précédentes, **caractérisée par le fait que** le pigment métallique est présent en une teneur allant de 0,1 % à 25 % en poids, par rapport au poids total de la composition, et mieux de 1 % à 10 % en poids.

16. Composition selon l'une quelconque des revendications précédentes, **caractérisée par le fait que** l'organopolysiloxane et le pigment métallique sont présents dans la composition en un rapport pondéral organopolysiloxane/pigment métallique allant de 0,1/100 à 5/100, et mieux de 0,5/100 à 1,5/100.

17. Composition selon l'une quelconque des revendications précédentes, **caractérisée par le fait que** la silice pyrogénée est une silice hydrophile.

18. Composition selon l'une quelconque des revendications précédentes, **caractérisée par le fait que** la silice pyrogénée est présente en une teneur allant de 0,1 % à 5 % en poids, par rapport au poids total de la composition, et mieux de 0,5 % à 2 % en poids.

19. Composition selon l'une quelconque des revendications précédentes, **caractérisée par le fait qu'**elle comprend, en outre, une argile en une teneur telle que le rapport pondéral argile/silice pyrogénée va de 0 à 0,5.

20. Composition selon l'une quelconque des revendications précédentes, **caractérisée par le fait qu'**elle comprend au moins un additif choisi dans le groupe formé par les agents épaississants, les agents d'étalement, les agents mouillants, les agents dispersants, les anti-mousses, les conservateurs, les filtres UV, les colorants, les pigments différents des pigments métalliques, les actifs, les tensioactifs, les agents hydratants, les parfums, les neutralisants, les stabilisants, les antioxydants.

21. Procédé cosmétique de maquillage des ongles, **caractérisé par le fait que** l'on applique sur les ongles une composition selon l'une quelconque des revendications 1 à 20.

22. Procédé cosmétique de maquillage des ongles, **caractérisé par le fait que** l'on applique sur les ongles :
- une première couche d'une première composition comprenant dans un milieu cosmétiquement acceptable un premier polymère filmogène,
- puis on applique sur au moins une partie de ladite première couche, une deuxième couche d'une deuxième composition comprenant au moins un deuxième polymère filmogène dans un milieu solvant organique, au moins une silice pyrogénée, au moins un pigment métallique et au moins un organopolysiloxane ayant en moyenne de 1,99 à 2,01 radicaux hydrocarbonés par atome de silicium, au moins 50 % des unités de l'organopolysiloxane étant des unités de formule RMeSiO dans laquelle R désigne un radical aliphatique ayant de 1 à 3 atomes de carbone, les autres unités restantes étant des unités de formule R'ₙSiO_{(2-n/2)} dans laquelle n a une valeur moyenne allant de 1 à 3 et R' désigne un radical hydrocarboné monovalent, une solution à 10 % en poids de l'organopolysiloxane dans le xylène ayant ayant une viscosité, à 25 °C, allant de 0,0002 à 0,01 m²/s,
- la première composition ne comprenant pas d'organopolysiloxane comme présent dans la deuxième composition.

23. Procédé selon la revendication 22, **caractérisé par le fait que** la solution d'organopolysiloxane à 10 % en poids dans le xylène a une viscosité allant de 0,0005 à 0,005 m²/s, et mieux de 0,001 m²/s à 0,0015 m²/s.

24. Procédé selon la revendication 22 ou 23, **caractérisée par le fait que** R désigne un radical méthyle.

25. Procédé selon l'une quelconque des revendications 22 à 24, **caractérisée par le fait que** l'organopolysiloxane est un polydiméthylsiloxane.

26. Procédé selon l'une quelconque des revendications 22 à 25, **caractérisée par le fait que** l'organopolysiloxane est présent en une teneur allant de 0,0001 % à 1,25 % en poids, par rapport au poids total de la composition, de préférence de 0,001 % à 0,5 % en poids, et mieux de 0,001 % à 0,25 % en poids.

27. Procédé selon l'une quelconque des revendications 22 à 26, **caractérisé par le fait que** le pigment métallique est choisi dans le groupe formé par la poudre d'aluminium, de cuivre, de zinc, de bronze, de nickel, de chrome, et leurs mélanges.

28. Procédé selon l'une quelconque des revendications 22 à 27, **caractérisé par le fait que** le pigment métallique est présent en une teneur allant de 0,1 % à 25 % en poids, par rapport au poids total de la deuxième composition, et mieux de 1 % à 10 % en poids.

29. Procédé selon l'une quelconque des revendications 22 à 28, **caractérisé par le fait que** l'organopolysiloxane et le pigment métallique sont présents dans la deuxième composition en un rapport pondéral organopolysiloxane/pigment métallique allant de 0,1/100 à 5/100, et mieux de 0,5/100 à 1,5/100.

30. Procédé selon l'une quelconque des revendications 22 à 29, **caractérisée par le fait que** la silice pyrogénée est une silice hydrophile.

31. Procédé selon l'une quelconque des revendications 22 à 30, **caractérisée par le fait que** la silice pyrogénée est présente en une teneur allant de 0,1 % à 5 % en poids, par rapport au poids total de la deuxième composition, et mieux de 0,5 % à 2 % en poids.

32. Procédé selon l'une quelconque des revendications 22 à 31, **caractérisé par le fait que** la première composition comprend un milieu solvant organique ou un milieu aqueux.

33. Procédé selon l'une quelconque des revendications 22 à 32, **caractérisé par le fait que** les premier et deuxième polymères filmogènes sont choisis, indifféremment l'un de l'autre, dans le groupe formé par les polymères radicalaires, les polycondensats et les polymères d'origine naturelle.

34. Procédé selon l'une quelconque des revendications 22 à 33, **caractérisé par le fait que** les premier et deuxième polymères filmogènes sont choisis, indifféremment l'un de l'autre, dans le groupe formé par les polymères vinyliques, les polyuréthanes, les polyesters, les polymères cellulosiques.

35. Procédé selon l'une quelconque des revendications 22 à 34, **caractérisé par le fait que** la teneur en respectivement premier et deuxième polymère filmogène va de 1 % à 60 % en poids, par rapport au poids total respectivement de la première et deuxième composition, et de préférence de 5 % à 45 % en poids.

36. Procédé selon l'une quelconque des revendications 22 à 35, **caractérisé par le fait que** le deuxième polymère comprend un ester de cellulose.

37. Procédé selon l'une quelconque des revendications 22 à 36, **caractérisé par le fait que** le deuxième polymère filmogène comprend un ester de cellulose choisi dans le groupe formé par l'acétobutyrate de cellulose et l'acétopropionate de cellulose.

38. Procédé selon la revendication 36 ou 37, **caractérisé par le fait que** l'ester de cellulose est présent en une teneur allant de 0,05 % à 30 % en poids, par rapport au poids total de la deuxième composition.

39. Procédé selon l'une quelconque des revendications 22 à 38, **caractérisé par le fait que** le deuxième polymère filmogène comprend de la nitrocellulose.

40. Procédé selon la revendication 39, **caractérisée par le fait que** la nitrocellulose est présente en une teneur allant de 0,05 % à 30 % en poids, par rapport au poids total de la composition.

41. Kit de maquillage comprenant :
- une première composition comprenant au moins un premier polymère filmogène dans un milieu cosmétiquement acceptable, et
- une deuxième composition comprenant au moins un deuxième polymère filmogène dans un milieu solvant organique, au moins une silice pyrogénée, au moins un pigment métallique et au moins un organopolysiloxane ayant en moyenne de 1,99 à 2,01 radicaux hydrocarbonés par atome de silicium, au moins 50 % des unités de l'organopolysiloxane dont des unités de formule RMeSiO dans laquelle R désigne un radical aliphatique ayant de 1 à 3 atomes de carbone, les autres unités restantes étant des unités de formule R'ₙSiO_{(2-n/2)} dans laquelle n a une valeur moyenne allant de 1 à 3 et R' désigne un radical hydrocarboné monovalent, une solution à 10 % en poids de l'organopolysiloxane dans le xylène ayant ayant une viscosité, à 25 °C, allant de 0,0002 m²/s à 0,01 m²/s,
- la première composition ne comprenant pas d'organopolysiloxane comme présent dans la deuxième composition.

42. Kit de maquillage selon la revendication 41, **caractérisé par le fait que** la solution d'organopolysiloxane à 10 % en poids dans le xylène a une viscosité allant de 0,0005 à 0,005 m²/s, et mieux de 0,001 m²/s à 0,0015 m²/s.

43. Kit de maquillage selon l'une des revendications 41 ou 42, **caractérisé par le fait que** R désigne un radical méthyle.

44. Kit de maquillage selon l'une quelconque des revendications 41 à 43, **caractérisé par le fait que** l'organopolysiloxane est un polydiméthylsiloxane.

45. Kit de maquillage selon l'une quelconque des revendications 41 à 44, **caractérisé par le fait que** l'organopolysiloxane est présent en une teneur allant de 0,0001 % à 1,25 % en poids, par rapport au poids total de la composition, de préférence de 0,001 % à 0,5 % en poids, et mieux de 0,001 % à 0,25 % en poids.

46. Kit de maquillage selon l'une quelconque des revendications 41 à 45, **caractérisé par le fait que** le pigment métallique est choisi dans le groupe formé par la poudre d'aluminium, de cuivre, de zinc, de bronze, de nickel, de chrome, et leurs mélanges.

47. Kit de maquillage selon l'une quelconque des revendications 41 à 46, **caractérisé par le fait que** le pigment métallique est présent en une teneur allant de 0,1 % à 25 % en poids, par rapport au poids total de la deuxième composition, et mieux de 1 % à 10 % en poids.

48. Kit de maquillage selon l'une quelconque des revendications 41 à 47, **caractérisé par le fait que** l'organopolysiloxane et le pigment métallique sont présents dans la deuxième composition en un rapport pondéral organopolysiloxane/pigment métallique allant de 0,1/100 à 5/100, et mieux de 0,5/100 à 1,5/100.

49. Kit de maquillage selon l'une quelconque des revendications 41 à 48, **caractérisé par le fait que** la silice pyrogénée est une silice pyrogénée hydrophile.

50. Kit de maquillage selon l'une quelconque des revendications 41 à 49, **caractérisé par le fait que** la silice pyrogénée est présente en une teneur allant de 0,1 % à 5 % en poids, par rapport au poids total de la deuxième composition, et mieux de 0,5 % à 2 % en poids.

51. Kit de maquillage selon l'une quelconque des revendications 41 à 50, **caractérisé par le fait que** la première composition comprend un milieu solvant organique ou un milieu aqueux.

52. Kit de maquillage selon l'une quelconque des revendications 41 à 51, **caractérisé par le fait que** les premier et deuxième polymères filmogènes sont choisis, indifféremment l'un de l'autre, dans le groupe formé par les polymères radicalaires, les polycondensats et les polymères d'origine naturelle.

53. Kit de maquillage selon l'une quelconque des revendications 41 à 52, **caractérisé par le fait que** les premier et deuxième polymères filmogènes sont choisis, indifféremment l'un de l'autre, dans le groupe formé par les polymères vinyliques, les polyuréthanes, les polyesters, les polymères cellulosiques.

54. Kit de maquillage selon l'une quelconque des revendications 41 à 53, **caractérisé par le fait que** la teneur en respectivement premier et deuxième polymère filmogène va de 1 % à 60 % en poids, par rapport au poids total respectivement de la première et deuxième composition, et de préférence de 5 % à 45 % en poids.

55. Kit de maquillage selon l'une quelconque des revendications 41 à 54, **caractérisé par le fait que** le deuxième polymère comprend un ester de cellulose.

56. Kit de maquillage selon l'une quelconque des revendications 41 à 55, **caractérisé par le fait que** le deuxième polymère filmogène comprend un ester de cellulose choisi dans le groupe formé par l'acétobutyrate de cellulose et l'acétopropionate de cellulose.

57. Kit de maquillage selon l'une quelconque des revendications 55 ou 56, **caractérisé par le fait que** l'ester de cellulose est présent en une teneur allant de 0,05 % à 30 % en poids, par rapport au poids total de la deuxième composition.

58. Kit de maquillage selon l'une quelconque des revendications 41 à 57, **caractérisé par le fait que** le deuxième polymère filmogène comprend de la nitrocellulose.

59. Kit de maquillage selon la revendication 58, **caractérisé par le fait que** la nitrocellulose est présente en une teneur allant de 0,05 % à 30 % en poids, par rapport au poids total de la composition.

## Patentansprüche

1. Zusammensetzung für Nagellacke, die in einem organischen Lösungsmittelmedium mindestens ein filmbildendes Polymer enthält, **dadurch gekennzeichnet, daß** sie mindestens eine pyrogene Kieselsäure, mindestens ein Metallpigment und mindestens ein Organopolysiloxan enthält, das im Mittel 1,99 bis 2,01 Kohlenwasserstoffgruppen pro Siliciumatom aufweist, wobei mindestens 50 % der Einheiten des Organopolysiloxans Einheiten der Formel RMeSiO sind, worin R eine aliphatische Gruppe mit 1 bis 3 Kohlenstoffatomen bedeutet, wobei die restlichen Einheiten Einheiten der Formel R'ₙSiO_{(2-n/2)} bedeuten, worin n ein Mittelwert von 1 bis 3 ist und R' eine einwertige Kohlenwasserstoffgruppe bedeutet, und wobei eine Lösung von 10 Gew.-% Organopolysiloxan in Xylol bei 25 °C eine Viskosität von 0,0002 bis 0,01 m²/s aufweist und das Gewichtsverhältnis von Kieselsäure/filmbildendes Polymer von 0,0116 verschieden ist.

2. Zusammensetzung nach Anspruch 1, **dadurch gekennzeichnet, daß** die Lösung von 10 Gew.-% Organopolysiloxan in Xylol eine Viskosität von 0,0005 bis 0,005 m²/s und besser 0,001 bis 0,0015 m²/s aufweist.

3. Zusammensetzung nach Anspruch 1 oder 2, **dadurch gekennzeichnet, daß** R Methyl bedeutet.

4. Zusammensetzung nach einem der vorhergehenden Ansprüche, **dadurch gekennzeichnet, daß** das Organopolysiloxan unter den Polydimethylsiloxanen, Polymethylethylsiloxanen, Dimethylsiloxan/Methylvinylsiloxan-Copolymeren und Dimethylsiloxan/Phenylmethylsiloxan-Copolymeren ausgewählt ist.

5. Zusammensetzung nach einem der vorhergehenden Ansprüche, **dadurch gekennzeichnet, daß** das Organopolysiloxan ein Polydimethylsiloxan ist.

6. Zusammensetzung nach einem der vorhergehenden Ansprüche, **dadurch gekennzeichnet, daß** das Organopolysiloxan in einem Mengenanteil von 0,0001 bis 1,25 Gew.-%, bezogen auf das Gesamtgewicht der Zusammensetzung, vorzugsweise 0,001 bis 0,5 Gew.-% und besser 0,001 bis 0,25 Gew.-% vorliegt.

7. Zusammensetzung nach einem der vorhergehenden Ansprüche, **dadurch gekennzeichnet, daß** das filmbildende Polymer unter den durch radikalische Polymerisation hergestellten Polymeren, den Polykondensaten und den Polymeren natürlicher Herkunft und vorzugsweise unter den Vinylpolymeren, Polyurethanen, Polyestern, und Cellulosepolymeren ausgewählt ist.

8. Zusammensetzung nach einem der vorhergehenden Ansprüche, **dadurch gekennzeichnet, daß** das filmbildende Polymer in einer Menge von 1 bis 60 Gew.-%, bezogen auf das Gesamtgewicht der Zusammensetzung, und besser 5 bis 45 Gew.-% vorliegt.

9. Zusammensetzung nach einem der vorhergehenden Ansprüche, **dadurch gekennzeichnet, daß** das filmbildende Polymer einen Celluloseester umfaßt.

10. Zusammensetzung nach einem der vorhergehenden Ansprüche, **dadurch gekennzeichnet, daß** das filmbildende Polymer einen Celluloseester umfaßt, der unter Celluloseacetobutyrat und Celluloseacetopropionat ausgewählt ist.

11. Zusammensetzung nach Anspruch 9 oder 10, **dadurch gekennzeichnet, daß** der Celluloseester in einem Mengenanteil von 0,05 bis 30 Gew.-%, bezogen auf das Gesamtgewicht der Zusammensetzung, vorliegt.

12. Zusammensetzung nach einem der vorhergehenden Ansprüche, **dadurch gekennzeichnet, daß** das filmbildende Polymer die Nitrocellulose umfaßt.

13. Zusammensetzung nach dem vorhergehenden Anspruch, **dadurch gekennzeichnet, daß** die Nitrocellulose in einem Mengenanteil von 0,05 bis 30 Gew.-%, bezogen auf das Gesamtgewicht der Zusammensetzung, vorliegt.

14. Zusammensetzung nach einem der vorhergehenden Ansprüche, **dadurch gekennzeichnet, daß** das Metallpigment unter den Pulvern von Aluminium, Kupfer, Zink, Bronze, Nickel, Chrom und deren Gemischen ausgewählt ist.

15. Zusammensetzung nach einem der vorhergehenden Ansprüche, **dadurch gekennzeichnet, daß** das Metallpigment in einem Mengenanteil von 0,1 bis 25 Gew.-%, bezogen auf das Gesamtgewicht der Zusammensetzung, und besser 1 bis 10 Gew.-% vorliegt.

16. Zusammensetzung nach einem der vorhergehenden Ansprüche, **dadurch gekennzeichnet, daß** das Organopolysiloxan und das Metallpigment in der Zusammensetzung in einem Gewichtsverhältnis Organopolysiloxan/Metallpigment von 0,1/100 bis 5/100 und besser 0,5/100 bis 1,5/100 vorliegt.

17. Zusammensetzung nach einem der vorhergehenden Ansprüche, **dadurch gekennzeichnet, daß** die pyrogene Kieselsäure eine hydrophile Kieselsäure ist.

18. Zusammensetzung nach einem der vorhergehenden Ansprüche, **dadurch gekennzeichnet, daß** die pyrogene Kieselsäure in einem Mengenanteil von 0,1 bis 5 Gew.-%, bezogen auf das Gesamtgewicht der Zusammensetzung, und besser 0,5 bis 2 Gew.-% vorliegt.

19. Zusammensetzung nach einem der vorhergehenden Ansprüche, **dadurch gekennzeichnet, daß** sie ferner einen Ton in einer solchen Menge enthält, daß das Gewichtsverhältnis Ton/pyrogene Kieselsäure im Bereich von 0 bis 0,5 liegt.

20. Zusammensetzung nach einem der vorhergehenden Ansprüche, **dadurch gekennzeichnet, daß** sie mindestens einen Zusatzstoff enthält, der ausgewählt ist unter: Verdickungsmitteln, Spreitmitteln, Netzmitteln, Dispergiermitteln, Antischaummitteln, Konservierungsmitteln, UV-Filtern, Farbmitteln, Pigmenten, die von den oben angegebenen Metallpigmenten verschieden sind, Wirkstoffen, grenzflächenaktiven Stoffen, Hydratisierungsmitteln, Parfums, Neutralisationsmitteln, Stabilisierungsmitteln und Antioxidantien.

21. Kosmetisches Verfahren zum Schminken der Nägel, **dadurch gekennzeichnet, daß** auf die Nägel eine Zusammensetzung nach einem der Ansprüche 1 bis 20 aufgetragen wird.

22. Kosmetisches Verfahren zum Schminken der Nägel, **dadurch gekennzeichnet, daß** auf die Nägel aufgetragen wird:
- eine erste Schicht einer ersten Zusammensetzung, die in einem kosmetisch akzeptablen Medium ein erstes filmbildendes Polymer enthält, und
- dann auf zumindest einem Teil der ersten Schicht eine zweite Schicht einer zweiten kosmetischen Zusammensetzung, die in einem organischen Lösungsmittelmedium mindestens ein zweites filmbildendes Polymer, mindestens eine pyrogene Kieselsäure, mindestens ein Metallpigment und mindestens ein Organopolysiloxan enthält, das im Mittel 1,99 bis 2,01 Kohlenwasserstoffgruppen pro Siliciumatom aufweist, wobei mindestens 50 % der Einheiten des Organopolysiloxans Einheiten der Formel RMeSiO sind, worin R eine aliphatische Gruppe mit 1 bis 3 Kohlenstoffatomen bedeutet, wobei die restlichen Einheiten Einheiten der Formel R'ₙSiO_{(2-n/2)} bedeuten, worin n ein Mittelwert von 1 bis 3 ist und R' eine einwertige Kohlenwasserstoffgruppe bedeutet, und wobei eine Lösung von 10 Gew.-% Organopolysiloxan in Xylol bei 25 °C eine Viskosität von 0,0002 bis 0,01 m²/s aufweist,
- wobei die erste Zusammensetzung kein Organopolysiloxan, wie es in der zweiten Zusammensetzung vorliegt, enthält.

23. Verfahren nach Anspruch 22, **dadurch gekennzeichnet, daß** die Lösung von 10 Gew.-% Organopolysiloxan in Xylol eine Viskosität von 0,0005 bis 0,005 m²/s und vorzugsweise 0,001 bis 0,0015 m²/s aufweist.

24. Verfahren nach Anspruch 22 oder 23, **dadurch gekennzeichnet, daß** R Methyl bedeutet.

25. Verfahren nach einem der Ansprüche 22 bis 24, **dadurch gekennzeichnet, daß** das Organopolysiloxan ein Polydimethylsiloxan ist.

26. Verfahren nach einem der Ansprüche 22 bis 25, **dadurch gekennzeichnet, daß** das Organopolysiloxan in einer Menge von 0,0001 bis 1,25 Gew.-%, bezogen auf das Gesamtgewicht der Zusammensetzung, vorzugsweise 0,001 bis 0,5 Gew.-% und besser 0,001 bis 0,25 Gew.-% vorliegt.

27. Verfahren nach einem der Ansprüche 22 bis 26, **dadurch gekennzeichnet, daß** das Metallpigment unter den Pulvern von Aluminium, Kupfer, Zink, Bronze, Nickel, Chrom und deren Gemischen ausgewählt ist.

28. Verfahren nach einem der Ansprüche 22 bis 27, **dadurch gekennzeichnet, daß** das Metallpigment in einem Mengenanteil von 0,1 bis 25 Gew.-%, bezogen auf das Gesamtgewicht der zweiten Zusammensetzung, und besser 1 bis 10 Gew.-% vorliegt.

29. Verfahren nach einem der Ansprüche 22 bis 28, **dadurch gekennzeichnet, daß** das Organopolysiloxan und das Metallpigment in der zweiten Zusammensetzung in einem Gewichtsverhältnis Organopolysiloxan/Metallpigment von 0,1/100 bis 5/100 und besser 0,5/100 bis 1,5/100 vorliegen.

30. Verfahren nach einem der Ansprüche 22 bis 29, **dadurch gekennzeichnet, daß** die pyrogene Kieselsäure eine hydrophile Kieselsäure ist.

31. Verfahren nach einem der Ansprüche 22 bis 30, **dadurch gekennzeichnet, daß** die pyrogene Kieselsäure in einem Mengenanteil von 0,1 bis 5 Gew.-%, bezogen auf das Gesamtgewicht der zweiten Zusammensetzung, und besser 0,5 bis 2 Gew.-% vorliegt.

32. Verfahren nach einem der Ansprüche 22 bis 31, **dadurch gekennzeichnet, daß** die erste Zusammensetzung ein organisches Lösungsmittelmedium oder ein wäßriges Medium enthält.

33. Verfahren nach einem der Ansprüche 22 bis 32, **dadurch gekennzeichnet, daß** die ersten filmbildenden Polymere und die zweiten filmbildenden Polymere unabhängig voneinander unter den durch radikalische Polymerisation hergestellten Polymeren, den Polykondensaten und den Polymeren natürlicher Herkunft ausgewählt sind.

34. Verfahren nach einem der Ansprüche 22 bis 33, **dadurch gekennzeichnet, daß** die ersten filmbildenden Polymere und die zweiten filmbildenden Polymere unabhängig voneinander unter den Vinylpolymeren, Polyurethanen, Polyestern und Cellulosepolymeren ausgewählt sind.

35. Verfahren nach einem der Ansprüche 22 bis 34, **dadurch gekennzeichnet, daß** der Mengenanteil des ersten filmbildenden Polymers bzw. des zweiten filmbildenden Polymers im Bereich von 1 bis 60 Gew.-%, bezogen auf das Gesamtgewicht der ersten bzw. zweiten Zusammensetzung, und vorzugsweise 5 bis 45 Gew.-% liegt.

36. Verfahren nach einem der Ansprüche 22 bis 35, **dadurch gekennzeichnet, daß** das zweite Polymer einen Celluloseester umfaßt.

37. Verfahren nach einem der Ansprüche 22 bis 36, **dadurch gekennzeichnet, daß** das zweite filmbildende Polymer einen Celluloseester umfaßt, der unter Celluloseacetobutyrat und Celluloseacetopropionat ausgewählt ist.

38. Verfahren nach Anspruch 36 oder 37, **dadurch gekennzeichnet, daß** der Celluloseester in einem Mengenanteil von 0,05 bis 30 Gew.-%, bezogen auf das Gesamtgewicht der zweiten Zusammensetzung, vorliegt.

39. Verfahren nach einem der Ansprüche 22 bis 38, **dadurch gekennzeichnet, daß** das zweite filmbildende Polymer die Nitrocellulose umfaßt.

40. Verfahren nach Anspruch 39, **dadurch gekennzeichnet, daß** die Nitrocellulose in einem Mengenanteil von 0,05 bis 30 Gew.-%, bezogen auf das Gesamtgewicht der Zusammensetzung, vorliegt.

41. Kit zum Schminken, der enthält:
- eine erste Zusammensetzung, die in einem kosmetisch akzeptablen Medium mindestens ein erstes filmbildendes Polymer enthält, und
- eine zweite Zusammensetzung, die in einem organischen Lösungsmittelmedium mindestens ein zweites filmbildendes Polymer, mindestens eine pyrogene Kieselsäure, mindestens ein Metallpigment und mindestens ein Organopolysiloxan enthält, das im Mittel 1,99 bis 2,01 Kohlenwasserstoffgruppen pro Siliciumatom aufweist, wobei mindestens 50 % der Einheiten des Organopolysiloxans Einheiten der Formel RMeSiO sind, worin R eine aliphatische Gruppe mit 1 bis 3 Kohlenstoffatomen bedeutet, wobei die restlichen Einheiten Einheiten der Formel R'ₙSiO_{(2-n/2)} bedeuten, worin n ein Mittelwert von 1 bis 3 ist und R' eine einwertige Kohlenwasserstoffgruppe bedeutet, und wobei eine Lösung von 10 Gew.-% Organopolysiloxan in Xylol bei 25 °C eine Viskosität von 0,0002 bis 0,01 m²/s aufweist,
- wobei die erste Zusammensetzung kein Organopolysiloxan, wie es in der zweiten Zusammensetzung vorliegt, enthält.

42. Kit zum Schminken nach Anspruch 41, **dadurch gekennzeichnet, daß** die Lösung von 10 Gew.-% Organopolysiloxan in Xylol eine Viskosität von 0,0005 bis 0,005 m²/s und besser 0,001 bis 0,0015 m²/s aufweist.

43. Kit zum Schminken nach einem der Ansprüche 41 oder 42, **dadurch gekennzeichnet, daß** R Methyl bedeutet.

44. Kit zum Schminken nach einem der Ansprüche 41 bis 43, **dadurch gekennzeichnet, daß** das Organopolysiloxan ein Polydimethylsiloxan ist.

45. Kit zum Schminken nach einem der Ansprüche 41 bis 44, **dadurch gekennzeichnet, daß** das Organopolysiloxan in einer Menge von 0,0001 bis 1,25 Gew.-%, bezogen auf das Gesamtgewicht der Zusammensetzung, vorzugsweise 0,001 bis 0,5 Gew.-% und besser 0,001 bis 0,25 Gew.-% vorliegt.

46. Kit zum Schminken nach einem der Ansprüche 41 bis 45, **dadurch gekennzeichnet, daß** das Metallpigment unter den Pulvern von Aluminium, Kupfer, Zink, Bronze, Nickel, Chrom und deren Gemischen ausgewählt ist.

47. Kit zum Schminken nach einem der Ansprüche 41 bis 46, **dadurch gekennzeichnet, daß** das Metallpigment in einem Mengenanteil von 0,1 bis 25 Gew.-%, bezogen auf das Gesamtgewicht der zweiten Zusammensetzung, und besser 1 bis 10 Gew.-% vorliegt.

48. Kit zum Schminken nach einem der Ansprüche 41 bis 47, **dadurch gekennzeichnet, daß** das Organopolysiloxan und das Metallpigment in der zweiten Zusammensetzung in einem Gewichtsverhältnis Organopolysiloxan/Metallpigment von 0,1/100 bis 5/100 und besser 0,5/100 bis 1,5/100 vorliegen.

49. Kit zum Schminken nach einem der Ansprüche 41 bis 48, **dadurch gekennzeichnet, daß** die pyrogene Kieselsäure eine hydrophile Kieselsäure ist.

50. Kit zum Schminken nach einem der Ansprüche 41 bis 49, **dadurch gekennzeichnet, daß** die pyrogene Kieselsäure in einem Mengenanteil von 0,1 bis 5 Gew.-%, bezogen auf das Gesamtgewicht der zweiten Zusammensetzung, und besser 0,5 bis 2 Gew.-% vorliegt.

51. Kit zum Schminken nach einem der Ansprüche 41 bis 50, **dadurch gekennzeichnet, daß** die erste Zusammensetzung ein organisches Lösungsmittelmedium oder ein wäßriges Medium enthält.

52. Kit zum Schminken nach einem der Ansprüche 41 bis 51, **dadurch gekennzeichnet, daß** die ersten filmbildenden Polymere und die zweiten filmbildenden Polymere unabhängig voneinander unter den durch radikalische Polymerisation hergestellten Polymeren, den Polykondensaten und den Polymeren natürlicher Herkunft ausgewählt sind.

53. Kit zum Schminken nach einem der Ansprüche 41 bis 52, **dadurch gekennzeichnet, daß** die ersten filmbildenden Polymere und die zweiten filmbildenden Polymere unabhängig voneinander unter den Vinylpolymeren, Polyurethanen, Polyestern und Cellulosepolymeren ausgewählt sind.

54. Kit zum Schminken nach einem der Ansprüche 41 bis 53, **dadurch gekennzeichnet, daß** der Mengenanteil des ersten filmbildenden Polymers bzw. des zweiten filmbildenden Polymers im Bereich von 1 bis 60 Gew.-%, bezogen auf das Gesamtgewicht der ersten bzw. zweiten Zusammensetzung, und vorzugsweise 5 bis 45 Gew.-% liegt.

55. Kit zum Schminken nach einem der Ansprüche 41 bis 54, **dadurch gekennzeichnet, daß** das zweite Polymer einen Celluloseester umfaßt.

56. Kit zum Schminken nach einem der Ansprüche 41 bis 55, **dadurch gekennzeichnet, daß** das zweite filmbildende Polymer einen Celluloseester umfaßt, der unter Celluloseacetobutyrat und Celluloseacetopropionat ausgewählt ist.

57. Kit zum Schminken nach einem der Ansprüche 55 oder 56, **dadurch gekennzeichnet, daß** der Celluloseester in einem Mengenanteil von 0,05 bis 30 Gew.-%, bezogen auf das Gesamtgewicht der Zusammensetzung, vorliegt.

58. Kit zum Schminken nach einem der Ansprüche 41 bis 57, **dadurch gekennzeichnet, daß** das zweite filmbildende Polymer die Nitrocellulose umfaßt.

59. Kit zum Schminken nach Anspruch 58, **dadurch gekennzeichnet, daß** die Nitrocellulose in einem Mengenanteil von 0,05 bis 30 Gew.-%, bezogen auf das Gesamtgewicht der Zusammensetzung, vorliegt.

## Claims

1. Nail varnish composition comprising, in an organic solvent medium, at least one film-forming polymer, **characterized in that** it comprises at least one pyrogenic silica, at least one metal pigment and at least one organopolysiloxane having on average from 1.99 to 2.01 hydrocarbon radicals per silicon atom, at least 50% of the units of the organopolysiloxane being units of formula RMeSiO in which R denotes an aliphatic radical having from 1 to 3 carbon atoms, the other remaining units being units of formula R'ₙSiO_{(2-n/2)} in which n has a mean value ranging from 1 to 3 and R' denotes a monovalent hydrocarbon radical, a 10% by weight solution of the organopolysiloxane in xylene having a viscosity at 25°C ranging from 0.0002 m²/s to 0.01 m²/s, the silica/film-forming polymer ratio by weight being other than 0.0116.

2. Composition according to Claim 1, **characterized in that** the 10% by weight solution of organopolysiloxane in xylene has a viscosity ranging from 0.0005 to 0.005 m²/s and better still from 0.001 m²/s to 0.0015 m²/s.

3. Composition according to Claim 1 or 2, **characterized in that** R denotes a methyl radical.

4. Composition according to any one of the preceding claims, **characterized in that** the organopolysiloxane is chosen from the group formed by polydimethylsiloxanes, polymethylethylsiloxanes, copolymers of dimethylsiloxane and of methylvinylsiloxane, and copolymers of dimethylsiloxane and of phenylmethylsiloxane.

5. Composition according to any one of the preceding claims, **characterized in that** the organopolysiloxane is a polydimethylsiloxane.

6. Composition according to any one of the preceding claims, **characterized in that** the organopolysiloxane is present at a content ranging from 0.0001% to 1.25% by weight with respect to the total weight of the composition, preferably from 0.001% to 0.5% by weight and better still from 0.001% to 0.25% by weight.

7. Composition according to any one of the preceding claims, **characterized in that** the film-forming polymer is chosen from the group formed by radical polymers, polycondensates and polymers of natural origin and preferably from the group formed by vinyl polymers, polyurethanes, polyesters and cellulose polymers.

8. Composition according to any one of the preceding claims, **characterized in that** the film-forming polymer is present at a content ranging from 1% to 60% by weight with respect to the total weight of the composition and better still from 5% to 45% by weight.

9. Composition according to any one of the preceding claims, **characterized in that** the film-forming polymer comprises a cellulose ester.

10. Composition according to any one of the preceding claims, **characterized in that** the film-forming polymer comprises a cellulose ester chosen from the group formed by cellulose acetobutyrate and cellulose acetopropionate.

11. Composition according to Claim 9 or 10, **characterized in that** the cellulose ester is present at a content ranging from 0.05% to 30% by weight with respect to the total weight of the composition.

12. Composition according to any one of the preceding claims, **characterized in that** the film-forming polymer comprises nitrocellulose.

13. Composition according to the preceding claim, **characterized in that** the nitrocellulose is present at a content ranging from 0.05% to 30% by weight with respect to the total weight of the composition.

14. Composition according to any one of the preceding claims, **characterized in that** the metal pigment is chosen from the group formed by aluminium powder, copper powder, zinc powder, bronze powder, nickel powder, chromium powder and their mixtures.

15. Composition according to any one of the preceding claims, **characterized in that** the metal pigment is present at a content ranging from 0.1% to 25% by weight with respect to the total weight of the composition and better still from 1% to 10% by weight.

16. Composition according to any one of the preceding claims, **characterized in that** the organopolysiloxane and the metal pigment are present in the composition in an organopolysiloxane/metal pigment ratio by weight ranging from 0.1/100 to 5/100 and better still from 0.5/100 to 1.5/100.

17. Composition according to any one of the preceding claims, **characterized in that** the pyrogenic silica is a hydrophilic silica.

18. Composition according to any one of the preceding claims, **characterized in that** the pyrogenic silica is present at a content ranging from 0.1% to 5% by weight with respect to the total weight of the composition and better still from 0.5% to 2% by weight.

19. Composition according to any one of the preceding claims, **characterized in that** it additionally comprises a clay at a content such that the clay/ pyrogenic silica ratio by weight ranges from 0 to 0.5.

20. Composition according to any one of the preceding claims, **characterized in that** it comprises at least one additive chosen from the group formed by thickening agents, spreading agents, wetting agents, dispersing agents, anti-foaming agents, preservatives, UV screening agents, dyes, pigments other than the metal pigments, active principles, surfactants, moisturizing agents, fragrances, neutralizing agents, stabilizing agents and antioxidants.

21. Cosmetic process for making up the nails, **characterized in that** a composition according to any one of Claims 1 to 20 is applied to the nails.

22. Cosmetic process for making up the nails, **characterized in that**,the nails have applied to them:
- a first layer of a first composition comprising, in a cosmetically acceptable medium, a first film-forming polymer,
- and then at least part of the said first layer has applied to it a second layer of a second composition comprising at least one second film-forming polymer in an organic solvent medium, at least one pyrogenic silica, at least one metal pigment and at least one organopolysiloxane having on average from 1.99 to 2.01 hydrocarbon radicals per silicon atom, at least 50% of the units of the organopolysiloxane being units of formula RMeSiO in which R denotes an aliphatic radical having from 1 to 3 carbon atoms, the other remaining units being units of formula R'ₙSiO_{(2-n/2)} in which n has a mean value ranging from 1 to 3 and R' denotes a monovalent hydrocarbon radical, a 10% by weight solution of the organopolysiloxane in xylene having a viscosity at 25°C ranging from 0.0002 to 0.01 m²/s,
- the first composition not comprising organopolysiloxane as present in the second composition.

23. Process according to Claim 22, **characterized in that** the 10% by weight solution of organopolysiloxane in xylene has a viscosity ranging from 0.0005 to 0.005 m²/s and better still from 0.001 m²/s to 0.0015 m²/s.

24. Process according to Claim 22 or 23, **characterized in that** R denotes a methyl radical.

25. Process according to any one of Claims 22 to 24, **characterized in that** the organopolysiloxane is a polydimethylsiloxane.

26. Process according to any one of Claims 22 to 25, **characterized in that** the organopolysiloxane is present at a content ranging from 0.0001% to 1.25% by weight with respect to the total weight of the composition, preferably from 0.001% to 0.5% by weight and better still from 0.001% to 0.25% by weight.

27. Process according to any one of Claims 22 to 26, **characterized in that** the metal pigment is chosen from the group formed by aluminium powder, copper powder, zinc powder, bronze powder, nickel powder, chromium powder and their mixtures.

28. Process according to any one of Claims 22 to 27, **characterized in that** the metal pigment is present at a content ranging from 0.1% to 25% by weight with respect to the total weight of the second composition and better still from 1% to 10% by weight.

29. Process according to any one of Claims 22 to 28, **characterized in that** the organopolysiloxane and the metal pigment are present in the second composition in an organopolysiloxane/metal pigment ratio by weight ranging from 0.1/100 to 5/100 and better still from 0.5/100 to 1.5/100.

30. Process according to any one of Claims 22 to 29, **characterized in that** the pyrogenic silica is a hydrophilic silica.

31. Process according to any one of Claims 22 to 30, **characterized in that** the pyrogenic silica is present at a content ranging from 0.1% to 5% by weight with respect to the total weight of the second composition and better still from 0.5% to 2% by weight.

32. Process according to any one of Claims 22 to 31, **characterized in that** the first composition comprises an organic solvent medium or an aqueous medium.

33. Process according to any one of Claims 22 to 32, **characterized in that** the first and second film-forming polymers are chosen, without distinction from one another, from the group formed by radical polymers, polycondensates and polymers of natural origin.

34. Process according to any one of Claims 22 to 33, **characterized in that** the first and second film-forming polymers are chosen, without distinction from one another, from the group formed by vinyl polymers, polyurethanes, polyesters and cellulose polymers.

35. Process according to any one of Claims 22 to 34, **characterized in that** the content of first and second film-forming polymer respectively ranges from 1% to 60% by weight with respect to the total weight of the first and second composition respectively and preferably from 5% to 45% by weight.

36. Process according to any one of Claims 22 to 35, **characterized in that** the second polymer comprises a cellulose ester.

37. Process according to any one of Claims 22 to 36, **characterized in that** the second film-forming polymer comprises a cellulose ester chosen from the group formed by cellulose acetobutyrate and cellulose acetopropionate.

38. Process according to Claim 36 or 37, **characterized in that** the cellulose ester is present at a content ranging from 0.05% to 30% by weight with respect to the total weight of the second composition.

39. Process according to any one of Claims 22 to 38, **characterized in that** the second film-forming polymer comprises nitrocellulose.

40. Process according to Claim 39, **characterized in that** the nitrocellulose is present at a content ranging from 0.05% to 30% by weight with respect to the total weight of the composition.

41. Make-up kit comprising:
- a first composition comprising at least one first film-forming polymer in a cosmetically acceptable medium, and
- a second composition comprising at least one second film-forming polymer in an organic solvent medium, at least one pyrogenic silica, at least one metal pigment and at least one organopolysiloxane having on average from 1.99 to 2.01 hydrocarbon radicals per silicon atom, at least 50% of the units of the organopolysiloxane being units of formula RMeSiO in which R denotes an aliphatic radical having from 1 to 3 carbon atoms, the other remaining units being units of formula R'ₙSiO_{(2-n/2)} in which n has a mean value ranging from 1 to 3 and R' denotes a monovalent hydrocarbon radical, a 10% by weight solution of the organopolysiloxane in xylene having a viscosity at 25°C ranging from 0.0002 m²/s to 0.01 m²/s,
- the first composition not comprising organopolysiloxane as present in the second composition.

42. Make-up kit according to Claim 41, **characterized in that** the 10% by weight solution of organopolysiloxane in xylene has a viscosity ranging from 0.0005 to 0.005 m²/s and better still from 0.001 m²/s to 0.0015 m²/s.

43. Make-up kit according to either of Claims 41 and 42, **characterized in that** R denotes a methyl radical.

44. Make-up kit according to any one of Claims 41 to 43, **characterized in that** the organopolysiloxane is a polydimethylsiloxane.

45. Make-up kit according to any one of Claims 41 to 44, **characterized in that** the organopolysiloxane is present at a content ranging from 0.0001% to 1.25% by weight with respect to the total weight of the composition, preferably from 0.001% to 0.5% by weight and better still from 0.001% to 0.25% by weight.

46. Make-up kit according to any one of Claims 41 to 45, **characterized in that** the metal pigment is chosen from the group formed by aluminium powder, copper powder, zinc powder, bronze powder, nickel powder, chromium powder and their mixtures.

47. Make-up kit according to any one of Claims 41 to 46, **characterized in that** the metal pigment is present at a content ranging from 0.1% to 25% by weight with respect to the total weight of the second composition and better still, from 1% to 10% by weight.

48. Make-up kit according to any one of Claims 41 to 47, **characterized in that** the organopolysiloxane and the metal pigment are present in the second composition in an organopolysiloxane/metal pigment ratio by weight ranging from 0.1/100 to 5/100 and better still from 0.5/100 to 1.5/100.

49. Make-up kit according to any one of Claims 41 to 48, **characterized in that** the pryogenic silica is a hydrophilic pyrogenic silica.

50. Make-up kit according to any one of Claims 41 to 49, **characterized in that** the pyrogenic silica is present at a content ranging from 0.1% to 5% by weight with respect to the total weight of the second composition and better still from 0.5% to 2% by weight.

51. Make-up kit according to any one of Claims 41 to 50, **characterized in that** the first composition comprises an organic solvent medium or an aqueous medium.

52. Make-up kit according to any one of Claims 41 to 51, **characterized in that** the first and second film-forming polymers are chosen, without distinction from one another, from the group formed by radical polymers, polycondensates and polymers of natural origin.

53. Make-up kit according to any one of Claims 41 to 52, **characterized in that** the first and second film-forming polymers are chosen, without distinction from one another, from the group formed by vinyl polymers, polyurethanes, polyesters and cellulose polymers.

54. Make-up kit according to any one of Claims 41 to 53, **characterized in that** the content of first and second film-forming polymer respectively ranges from 1% to 60% by weight with respect to the total weight of the first and second composition respectively and preferably from 5% to 45% by weight.

55. Make-up kit according to any one of Claims 41 to 54, **characterized in that** the second polymer comprises a cellulose ester.

56. Make-up kit according to any one of Claims 41 to 55, **characterized in that** the second film-forming polymer comprises a cellulose ester chosen from the group formed by cellulose acetobutyrate and cellulose acetopropionate.

57. Make-up kit according to either one of Claims 55 and 56, **characterized in that** the cellulose ester is present at a content ranging from 0.05% to 30% by weight with respect to the total weight of the second composition.

58. Make-up kit according to any one of Claims 41 to 57, **characterized in that** the second film-forming polymer comprises nitrocellulose.

59. Make-up kit according to Claim 58, **characterized in that** the nitrocellulose is present at a content ranging from 0.05% to 30% by weight with respect to the total weight of the composition.
